# EUROPEAN PATENT SPECIFICATION

(11) **EP 4 299 562 B1**
(45) Date of publication and mention of the grant of the patent: **20.05.2026**
(21) Application number: 22760044.2
(22) Date of filing: 23.02.2022
(51) Int. Cl.: C07D 213/73, G01N 33/58, G01N 33/543, C09K 11/06, G01N 21/76, G01N 27/28, C09K 11/07, G01N 21/66

(54) **NOVEL ELECTROCHEMILUMINESCENCE CO-REACTANT, AND ELECTROCHEMILUMINESCENCE SYSTEM COMPRISING SAME**
NEUARTIGER CO-REAKTAND FÜR ELEKTROCHEMILUMINESZENZ UND ELEKTROCHEMILUMINESZENZSYSTEM DAMIT
NOUVEAU CO-RÉACTIF D'ÉLECTROCHIMIOLUMINESCENCE ET SYSTÈME D'ÉLECTROCHIMIOLUMINESCENCE LE COMPRENANT

(30) Priority: 26.02.2021 KR 20210026210; 08.02.2022 KR 20220016178
(43) Date of publication of application: 03.01.2024
(73) Proprietor: Elips Diagnostics Inc., Seoul 06978 (KR)
(72) Inventor: SHIN, Ik-Soo, Seoul 06978 (KR)
(74) Representative: Leonard, Thomas Charles
(86) International application number: PCT/KR2022/002654
(87) International publication number: WO 2022/182128

(56) References cited:
- WO-A1-2020/227016
- KR-A- 20190 083 619
- US-A1- 2009 246 076
- US-A1- 2018 238 882
- US-A1- 2020 191 699
- ANITHA DEVADOSS ET AL: "Electrochemiluminescent Metallopolymer-Nanoparticle Composites: Nanoparticle Size Effects", ANALYTICAL CHEMISTRY, vol. 83, no. 6, 15 March 2011 (2011-03-15), pages 2383 - 2387, XP055637286, DOI: 10.1021/ac102697c
- ANITHA DEVADOSS ET AL: "Highly sensitive detection of NADH using electrochemiluminescent nanocomposites", ELECTROCHEMISTRY COMMUNICATIONS, ELSEVIER AMSTERDAM, NL, vol. 19, 2 March 2012 (2012-03-02), pages 43 - 45, XP028511301, ISSN: 1388-2481, [retrieved on 20120311], DOI: 10.1016/J.ELECOM.2012.03.003
- ZANUT ALESSANDRA ET AL: "Abstract", NATURE COMMUNICATIONS, vol. 11, no. 1, 1 December 2020 (2020-12-01), XP055811549, DOI: 10.1038/s41467-020-16476-2

## Description

### TECHNICAL FIELD

The present invention relates to a novel use of a compound as an electrochemiluminescence (ECL) co-reactant, and use of an electrochemiluminescence system including the same. The electrochemiluminescence co-reactant including a compound represented by Chemical Formula (I), or a pharmaceutically acceptable salt thereof, can be widely and generally used in immunoassays and diagnostic devices based thereon which use an electrochemiluminescent label of a specific luminescent species (a polycyclic aromatic hydrocarbon compound, a metal complex compound, a quantum dot, or nanoparticles, and the like and have excellent the detection signal and improved voltage application condition when measuring electrochemiluminescence.

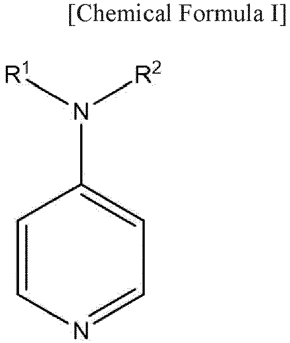

In Chemical Formula I, wherein each R¹ and R² may be the same as or different from each other, and represent one selected from the group consisting of: a hydrogen atom; a halogen atom; a C₁-C₆ straight-chain, branched, or cyclic alkyl group; a C₁-C₆ alkoxy group; and a C₁-C₆ haloalkyl group.

### BACKGROUND ART

Research continues for rapid, highly specific, sensitive and accurate methods of detecting and quantifying chemical, biochemical and biological materials. Improvements in analytical performance, such as sensitivity, are considered important because the amount of specific analytes in common biological samples is considerably low.

One approach to improving analytical sensitivity is to take advantage of methods available for highly sensitive photodetection (for example: photomultiplier tubes). In this regard, the use of luminescent indicator molecules is considered a very important factor. For example, a luminescent label associated with an analyte of interest (or a binding factor for the analyte of interest) can be used to quantitatively detect the presence of the analyte (or a binding partner).

The amount of analyte can be quantitatively determined when the analyte participates in a reaction that induces the regulation of luminescence as described below. Specifically, i) an analyte may react with another species to regulate the luminescent properties of the second species, ii) the analyte may undergo chemical modifications that regulate the luminescent properties of the analyte itself, iii) the analyte may be a catalyst (for example, an enzyme) that induces the reactions of other species, and iv) the analyte may participate in a reaction that produces a species, and then participate in subsequent reactions that induce luminescence regulation.

Methods for detecting luminescent indicator molecules include photoluminescence, chemiluminescence, and electrochemiluminescence (ECL).

Among various light detection systems, with an electrochemiluminescence (ECL) system, it is possible to construct an inexpensive and compact diagnostic system and minimize signal interference caused by background signals from other interferers because a light source, which has a large volume and is expensive, is not required.

More specifically, electrochemiluminescence (ECL) is an electrical light generation phenomenon discovered in about 1960, and such a phenomenon is a phenomenon which i) induces an oxidation reaction of a specific luminescence material through the application of voltage, ii) allows an intermediate reactant produced thereafter to change into a final product in an excited state through a secondary chemical reaction, and then iii) generates light while the excited state is converted to the ground state. Such ECL is used as a detection method in high-end medical immunodiagnostic devices.

As a form of utilization, a COBASⓡ diagnostic device using the Elecsys method from Roche and a Mesoscale Discovery ECL Systems series are the only immunodiagnostic devices based on electrochemiluminescence (ECL), and dominate the high-end immunodiagnostic device market around the world. These methods generate ECL using a ruthenium compound [Ru(bpy)₃²⁺] as a luminophore and tripropylamine (TPA) as a co-reactant, and have, as a problem, a disadvantage in that tripropylamine, which is a co-reactant, is hydrophobic, and thus, the luminescence efficiency is low under platinum and gold electrode conditions. Furthermore, in the 60-year history of electrochemiluminescence, there is still no co-reactant that exhibits greater luminescence efficiency than tripropylamine.

Specifically, tripropylamine (TPrA) is generally used as a co-reactant because it allows efficient electrochemiluminescence (ECL) in organic as well as aqueous media and even at physiological pH 7.4. However, TPrA has a disadvantage in that it has volatile toxicity and must be used at high concentrations (generally up to 100 mM) to obtain high electrochemiluminescence (ECL) signals. Further, TPrA has the inconvenience of requiring a highly concentrated buffer solution to prepare a solution because it has a slow electrochemical oxidation rate, limits electrochemiluminescence (ECL) efficiency, and is basic. In addition, TPrA has a disadvantage in that the deviation between the same detection signals is relatively large, and it chemically reacts with carbon dioxide in air.

Furthermore, the electrochemiluminescence (ECL) efficiency of ruthenium pyridine [Ru(bpy)₃²⁺] and tripropylamine (TPrA) is dependent on electrode materials. Platinum (Pt) and gold (Au) electrodes can be covered with an oxide layer of the positive electrode in a potential region where electrochemiluminescence occurred, which was found to suppress the direct oxidation of tripropylamine, resulting in an decrease in electrochemiluminescence intensity. In contrast, a polished glassy carbon (GC) electrode has a relatively fast electrochemical oxidation rate of tripropylamine, which results in a greater amount of tripropylamine oxidized on the electrode surface and a much greater emission intensity.

Based on this background, the present inventors have attempted to develop a new electrochemiluminescence co-reactant capable of improving the emission intensity and detection reproducibility of electrochemiluminescence (ECL) systems.

Compounds having a chemical structure according to Chemical Formula I of the claimed invention are known in the prior art, however they have not been used as electrochemiluminescence co-reactants (see e.g. A. Devadoss et al, Anal. Chem, 2011, 83, 2383 and A. Devadoss et al, Electrochem. commun., 2012, 19, 43).

### DETAILED DESCRIPTION

### Technical Problem

In regard to electrochemiluminescence (ECL), which is widely applicable to blood glucose, cholesterol sensors, molecular diagnostics, or antibody detection by immunoassay, the present inventors found that in the case of a pyridine derivative represented by Chemical Formula I, the luminescence intensity is excellent in an electrochemiluminescence system labeled with ruthenium pyridine while attempting to develop a novel co-reactant which has a fast reaction, and is less affected by electrode materials and excellent in luminescence efficiency by replacing the existing tripropylamine, which is mainly used as a co-reactant, thereby completing the present invention.

Therefore, an object of the present invention is to provide an electrochemiluminescence (ECL) co-reactant including a compound represented by Chemical Formula I, or a pharmaceutically acceptable salt thereof.

Further, another object of the present invention is to provide an electrochemiluminescence system which excites the co-reactant; and an electrochemiluminescent label.

In addition, still another object of the present invention is to provide a detection method using the electrochemiluminescence system.

Furthermore, yet another object of the present invention is to provide a kit including the co-reactant for electrochemiluminescence immunoassay or molecular diagnosis.

### Technical Solution

To achieve the above objects, the present invention provides for the use of a compound represented by the following Chemical Formula I, or a pharmaceutically acceptable salt thereof, as an electrochemiluminescence (ECL) co-reactant.

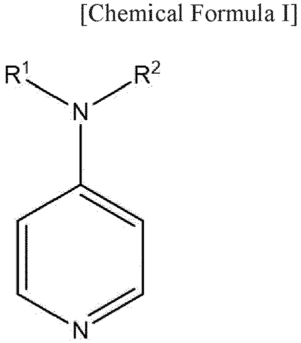

In Chemical Formula I, wherein each R¹ and R² may be the same as or different from each other, and represent one selected from the group consisting of: a hydrogen atom; a halogen atom; a C₁-C₆ straight-chain, branched, or cyclic alkyl group; a C₁-C₆ alkoxy group; and a C₁-C₆ haloalkyl group.

Further, the present invention provides for the use of an electrochemiluminescence system including: an electrochemical cell filled with an electrolyte solution including an electrochemiluminescence (ECL) co-reactant including the compound represented by Chemical Formula I, or a pharmaceutically acceptable salt thereof and an electrochemiluminescent label; and a photodetector connected to the electrochemical cell; wherein the use of the electrochemiluminescence system comprises using the compound or pharmaceutically acceptable salt as an electrochemiluminescence co-reactant.

Further, the present invention provides a detection method of an electrochemiluminescence system, the method including: (a) putting a sample into an electrochemiluminescence system including electrochemiluminescence (ECL) co-reactant including the compound represented by Chemical Formula I or a pharmaceutically acceptable salt thereof, and an electrochemiluminescent label and reacting the resulting mixture, wherein a compound or pharmaceutically acceptable salt as defined in any one of claims 1 to 3 is used as an electrochemiluminescence co-reactant; and (b) measuring the electrochemiluminescence intensity (ECL intensity) according to the input potential (or voltage) of the reaction sample of Step (a) using an electrochemiluminescence-based detector and detecting an optical signal.

In addition, the present invention provides for the use of a kit for electrochemiluminescence immunoassay or molecular diagnosis, the kit including the compound represented by Chemical Formula I or a pharmaceutically acceptable salt thereof and an electrochemiluminescent label; wherein the use of the kit comprises using the compound or pharmaceutically acceptable salt as an electrochemiluminescence co-reactant.

### Advantageous Effects

The pyridine derivative according to the present invention enables rapid and accurate detection with an electrochemiluminescent signal and thus replace tripropylamine (TPrA), which is a co-reactant in the related art.

Specifically, the pyridine derivative according to the present invention can provide wide application potential in various bioanalyses such as immunoassay because it has excellent handleability as a solid compound, can improve the potential (or voltage) conditions for luminescence, and can improve luminescence efficiency when used at a low concentration.

### BRIEF DESCRIPTION OF DRAWINGS

FIG. 1 illustrates a schematic view of an electrochemiluminescence system including a potentiostat and a photomultiplier tube, as an exemplary embodiment of the uses according to the present invention.
FIG. 2 shows the results of measuring a cyclic voltammogram (CV) in an electrochemiluminescence system including 5 mM co-reactant and 1 µM [Ru(bpy)₃]²⁺ ruthenium pyridine in 1X PBS (pH 7.4)(Scan rate: 0.1 V/s, WE: GC, CE: Pt, RE: Ag/AgCl).
FIG. 3 shows the results of measuring ECL intensity in an electrochemiluminescence system including 5 mM co-reactant and 1 µM [Ru(bpy)₃]²⁺ ruthenium pyridine in 1X PBS (pH 7.4)(Scan rate: 0.1 V/s, WE: GC, CE: Pt, RE: Ag/AgCl).
FIG. 4 shows the results of measuring the cyclic voltammogram (CV) in an electrochemiluminescence system including 5 mM co-reactant and 1 µM [Ru(bpy)₃]²⁺ in 1X PBS (pH 7.4)(Scan rate: 0.1 V/s, WE: Pt, CE: Pt, RE: Ag/AgCl).
FIG. 5 shows the results of measuring ECL intensity in an electrochemiluminescence system including 5 mM co-reactant and 1 µM [Ru(bpy)₃]²⁺ in 1X PBS (pH 7.4)(Scan rate: 0.1 V/s, WE: Pt, CE: Pt, RE: Ag/AgCl).
FIG. 6 is a plot showing the dependence of ECL intensity of 1 µM [Ru(bpy)₃]²⁺ ruthenium pyridine on the concentration of 4-DMAP and TPrA (1 to 100 mM) in 1X PBS (pH 7.4). The potential was increased stepwise from 0 V to 1.6 V (WE: Pt, CE : Pt, RE : Ag/AgCl).
FIG. 7 is a plot showing the dependence of ECL intensity of 10 µM [Ru(bpy)₃]²⁺ ruthenium pyridine on the concentration of 4-DMAP and TPrA (1 to 100 mM) in 1X PBS (pH 7.4). The potential was increased stepwise from 0 V to 1.6 V. (WE : Pt, CE : Pt, RE : Ag/AgCl).
FIG. 8 shows the results of measuring ECL intensity under various pH conditions using [Ru(bpy)₃]²⁺ ruthenium pyridine (1 µM and 10 µM) (WE : Pt, CE : Pt, RE : Ag/AgCl).
FIG. 9 is a set of graphs illustrating the comparison of chemiluminescence intensities when 4-DMAP and TPrA are used as co-reactants in an acetonitrile (ACN) solution, but glassy carbon (A), platinum (B) and gold (C) are used as working electrodes (CE : Pt, RE : Ag/AgCl).
FIG. 10 is a set of graphs illustrating a linear sweep voltammogram and electrochemiluminescence intensity when glassy carbon is used as a working electrode under the circumstances of using 4-DMAP as a co-reactant in an acetonitrile (ACN) solution (CE: Pt, RE: Ag/Ag⁺ (3 M AgNO₃), scan rate: 0.1 V/s).
FIG. 11 shows the results of obtaining the difference in electrochemiluminescence signals generated by changing the concentration of a Ru(bpy)₃²⁺ luminophore when 7 mM 4-DMAP and 7 mM tripropylamine were each used as co-reactants in the form of a calibration curve.
FIG. 12 shows the results of performing electrochemiluminescence immunodiagnosis on the "severe acute respiratory syndrome (SARS-CoV-2) neutralizing antibody (anti-SARS-CoV-2)" present in 10 saliva samples inoculated with a vaccine using 4-DMAP or tripropylamine as a co-reactant.

### BEST MODES OF THE INVENTION

Electrochemiluminescence is a luminescence process which occurs while a compound generated at an electrode undergoes an electron transfer reaction with high energy to form an excited state that emits light. Luminescent labeling reagents used for electrochemiluminescence include transition metal complex compounds, luminescent organic semiconductors, quantum dot materials, perovskite nanoparticles, metal nanoparticles or carbon nanoparticles. These organic and inorganic luminescent labels have been used for a wide range of bioanalysis to date.

The oxidation reaction, which is the basic principle of electrochemiluminescence, is the loss of electrons at the surface of an electrode by a luminescent substrate and a composition during the reaction process. An electron donor loses a hydrogen ion (H⁺) to become a strong reducing agent which reduces a luminescent substrate in the excited state, and thereafter, the luminescent substrate emits photons to return to the ground state. This process is repeatedly performed at the surface of the electrode, and photons are generally emitted continuously to keep the substrate concentration constant.

As an example, there is an electrochemiluminescence system of ruthenium pyridine and tripropylamine. The electrochemiluminescence reaction is a specific chemiluminescence reaction induced by electrochemistry on the surface of the electrode. A conjugate formed of antigen-antibody complexes and ruthenium pyridine conjugates are excited by electrochemistry in the presence of tripropylamine, and a redox reaction occurs to release photons, which can be sensed by a photomultiplier tube. This process is repeatedly performed to produce many photons, which amplify the optical signal. In general, labels used in electrochemiluminescence assays may bind to antibody or antigen molecules with different chemical structures to produce labeled antibodies or antigens.

In currently available electrochemiluminescence methods, tripropylamine (TPrA) is usually used as a co-reactant. The disadvantages of TPrA are that it is difficult to handle TPrA in a liquid phase, and TPrA is slow to react, requires high concentrations, is greatly affected by electrode materials, has limited luminescence efficiency, and has the limitations of toxicity and instability.

The present inventors confirmed that when a solid compound 4-dimethylaminopyridine (4-DMAP) is used as a co-reactant for electrochemiluminescence, it participates very quickly in the luminescence reaction and has excellent luminescence efficiency.

Therefore, the present invention provides for the use of a compound represented by Chemical Formula I**,** or a pharmaceutically acceptable salt thereof as an electrochemiluminescence (ECL) co-reactant.

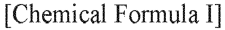

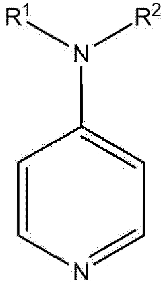

In Chemical Formula I, each R¹ and R² may be the same as or different from each other, and represent one selected from the group consisting of: a hydrogen atom; a halogen atom; a C₁-C₆ straight-chain, branched, or cyclic alkyl group; a C₁-C₆ alkoxy group; and a C₁-C₆ haloalkyl group.

In Chemical Formula I, each R¹ and R² may be the same as or different from each other, and represent one selected from the group consisting of: a hydrogen atom; a halogen atom; a C₁-C₄ straight-chain, branched, or cyclic alkyl group; a C₁-C₄ alkoxy group; and a C₁-C₄ haloalkyl group.

Preferably, each R¹ and R² may be the same as or different from each other, and may be a C₁-C₄ straight-chain or branched alkyl group; or a C₁ to C₄ haloalkyl group.

More preferably, Chemical Formula I may be 4-dimethylaminopyridine.

Further, the present invention provides for the use of an electrochemiluminescence system including: an electrochemical cell filled with an electrolyte solution including a compound represented by Chemical Formula I, or a pharmaceutically acceptable salt thereof, and an electrochemiluminescent label; and a photodetector connected to the electrochemical cell; wherein the use of the electrochemiluminescence system comprises using the compound or pharmaceutically acceptable salt as an electrochemiluminescence co-reactant.

The co-reactant is a compound having the structure of Chemical Formula I described above.

The electrochemiluminescent label may be one or more selected from the group consisting of a transition metal complex compound, a luminescent organic semiconductor, a quantum dot material, perovskite nanoparticles, metal nanoparticles and carbon nanoparticles, but is not limited thereto.

Specifically, as the transition metal compound, it is possible to use one or more selected from the group consisting of ruthenium (Ru), iridium (Ir), rhenium (Re), platinum (Pt), osmium (Os), copper (Cu), and iron (Fe).

Specifically, the ionic transition metal complex compound may include one or more selected from the group consisting of tris(2,2'-bipyridine)ruthenium(II)bis(hexafluorophosphate) [Ru(bpy)₃(PF₆)₂], tris(4,7-diphenyl-1,10-phenanthroline)ruthenium(II) bis(hexafluorophosphate) [Ru(dp-phen)₃(PF₆)₂], bis(2-phenylpyridine)(2,2'-dipyridine)iridium(III) (hexafluorophosphate) [Ir(ppy)₂(bpy)PF₆], bis(2-phenylpyridine)(4,4'-di-tert-butyl-2,2'-dipyridyl)iridium(III) (hexafluorophosphate) [Ir(dtbbpy)(ppy)₂PF₆], 4'-di-tert-butyl-2,2'-dipyridyl-bis[2-(2',4'-difluorophenyl)pyridine]iridium(III)(hexafluorophosphate) [Ir(ppy-F₂)₂(dtbbpy)PF₆], iridium bis[5-(trifluoromethyl)-2-(4-(trifluoromethyl)phenyl)pyridine] picolinate [Ir(ppy-(CF₃)₂)₂(pico)], tris[2-(p-tolyl)pyridine]iridium(III) [Ir(mppy)₃], 1,10-[phenanthroline]rhenium(I)(hexafluorophosphate) [Re(phen)PF₆], platinum(II) coproporphyrin [PtCP] and tris(2,2'-bipyridine)osmium(II)(hexafluorophosphate) [Os(bpy)₃(PF₆)₂], but is not limited thereto.

Specifically, the luminescent organic semiconductor may include a conjugated organic semiconductor capable of emitting light, such as a luminescent single molecule or a polymer. Specifically, the luminescent organic semiconductor may include one or more selected from the group consisting of luminol and rubrene and derivatives thereof, anthracene and derivatives thereof, pyrene and derivatives thereof, decycloxyphenyl substituted poly(1,4-phenylene vinylene) [super yellow], poly(2-methoxy-5-(2-ethylhexyloxy)-1,4-phenylenevinylene [MEH-PPV], poly(2-methoxy-5-(3',7'-dimethyloctyloxy)-1,4-phenylenevinylene) [MEMO-PPV], and poly(9,9-dioctylfluorene-alt-benzothiadiazole [F8BT], but is not limited thereto.

The quantum dot material may include an inorganic compound of Group 13-15 or Group 12-15 elements. Specifically, the quantum dot material including the inorganic compound may include one or more selected from the group consisting of cadmium selenide (CdSe), cadmium sulfide (CdS), zinc selenide (ZnSe), indium phosphide (InP), lead sulfide (PbS) and lead selenide (PbSe), but is not limited thereto.

The perovskite nanoparticles may include halide-based perovskites. Specifically, the halide-based perovskite may be represented by a chemical formula of ABX₃, A₂BX₆ or A₃B₂X₉. In this case, A may be an organic or inorganic cation, B may be a metal cation, and X may be a halide anion.

The metal nanoparticles may include metal atom clusters with a dimension of 1 nm or less, which exhibit discrete energy levels. Specifically, the metal nanoparticles may include gold (Au) nanoparticles, silver (Ag), copper (Cu), or silver (Ag)-gold (Au) bimetallic nanoparticles.

The carbon particles may include a graphene quantum dot (GQD) or a carbon quantum dot (CQD), but are not limited thereto.

In addition, the electrolyte solution may include a liquid electrolyte including a salt, water and an organic solvent; a solid electrolyte in which a salt is dissolved in a polymer; a gel-type electrolyte including a polymer, a salt, water, and an organic solvent; or an ionic gel electrolytes including a block copolymer and an ionic liquid, but is not limited thereto. The salt is an organic/inorganic ionic compound salt, and may include any one or a mixture of two or more selected from the group of phosphate, nitrate, sulfate, lithium salt, sodium salt, potassium salt, calcium salt, magnesium salt, ammonium salt, and the like, but is not limited thereto.

Specifically, the electrolyte solution used in the electrochemiluminescence system of the present invention may be a solution including water or an organic solvent of one or more selected from the group consisting of phosphate buffer saline (PBS), a Tris buffer solution, acetonitrile (ACN), dichloromethane, ethanol, methanol, tetrahydrofuran (THF), dimethyl sulfoxide (DMSO), dimethylformamide (DMF), ethylene carbonate (EC) and propylene carbonate (PC), but is not limited thereto.

The electrolyte solution may have a pH of 5 to 12, preferably 7.4 to 10.

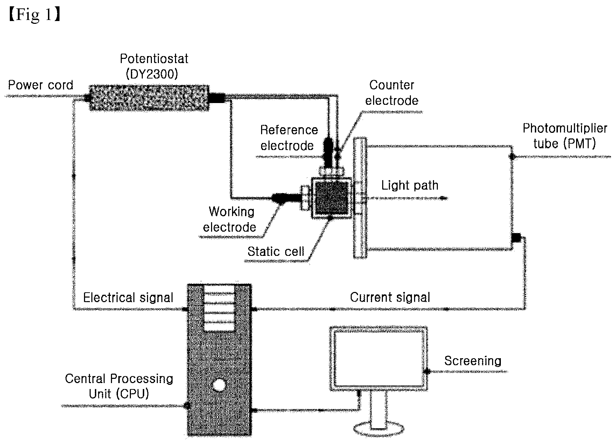

FIG. 1 shows an exemplary embodiment of an electrochemiluminescence system for performing electrochemiluminescence measurements in the uses according to the present invention, and the electrochemiluminescence system includes an electrochemical cell including an electrolyte solution, a potentiostat and a photomultiplier tube (PMT). In this case, the photomultiplier tube (PMT) is connected to a potentiostat and driven at the same time. Therefore, an electrochemiluminescence reaction may be caused by inducing a reaction in an electrochemical cell using a potentiostat for electrochemiluminescence measurement. For electrochemiluminescence measurement, a potentiostat and electrochemiluminescence measurement software may be run to measure the luminescence intensity of electrochemiluminescence, and the like.

The electrode that constitutes the electrochemical cell may include a working electrode, a reference electrode, a working electrode, and a counter electrode, but is not limited thereto.

The working electrode may be an electrode of one or more selected from the group consisting of carbon, platinum (Pt), gold (Au), silver (Ag), nickel (Ni), stainless steel, palladium, tin, indium and silicon elements, but is not necessarily limited thereto.

The counter electrode may be an electrode of one or more selected from the group consisting of carbon, platinum (Pt), gold (Au), silver (Ag), nickel (Ni), stainless steel, palladium, tin, indium and silicon elements, but is not necessarily limited thereto.

The reference electrode may be one or more selected from the group consisting of a silver (Ag)-based Ag pseudo-reference electrode, a Ag/AgCl electrode, a Ag/AgNO₃ electrode, a mercury (Hg) calomel electrode, a Hg/HgO electrode, and a Hg₂SO₄ electrode, but is not limited thereto.

In addition, the present invention provides a detection method of an electrochemiluminescence system, the method including: (a) putting an electrolyte solution including a sample into an electrochemical cell in the electrochemiluminescence system defined herein, and reacting the resulting mixture, wherein a compound represented by Chemical Formula I, or a pharmaceutically acceptable salt thereof, is used as an electrochemiluminescence co-reactant; and (b) measuring the electrochemiluminescence intensity (ECL intensity) according to the input voltage of the reaction sample of Step (a) using an electrochemiluminescence-based detector and detecting an optical signal.

Step (a) is a step of putting a sample into an electrolyte solution including the co-reactant and an electrochemiluminescence label, ruthenium pyridine, and reacting the resulting mixture, and the sample may be serum, urine, or tissue fluid, but is not limited thereto.

Step (b) is a step of measuring the electrochemiluminescence intensity (ECL intensity) according to the input potential (or voltage) of the reaction sample of Step (a) using an electrochemiluminescence-based photodetector. There are various types of photodetectors which are devices for measuring such luminescence. As an example, the photodetector may be one or more selected from the group consisting of a photodiode based on silicon, germanium, germanium-phosphide, indium-gallium-arsenide, and lead-sulfide; a photomultiplier tube (PMT); a charge coupled device (CCD); an electron-multiplying charge coupled device (EMCCD); and a scientific complementary metal-oxide-semiconductor (sCMOS), but is not limited thereto.

Furthermore, the present invention may provide an electrochemiluminescence immunoassay detection method through an electrochemical method. Therefore, the present invention provides for the use of a kit for electrochemiluminescence immunoassay or molecular diagnosis, the kit including the compound represented by Chemical Formula I or a pharmaceutically acceptable salt thereof and an electrochemiluminescent label; wherein the use of the kit comprises using the compound or pharmaceutically acceptable salt as an electrochemiluminescence co-reactant.

Specifically, the kit for electrochemiluminescence immunoassay or molecular diagnosis of the present invention may include an electrolyte solution, and the electrolyte solution may be a solution including water or an organic solvent of one or more selected from the group consisting of phosphate buffer saline (PBS), a Tris buffer solution, acetonitrile (ACN), dichloromethane, ethanol, methanol, tetrahydrofuran (THF), dimethyl sulfoxide (DMSO), dimethylformamide (DMF), ethylene carbonate (EC) and propylene carbonate (PC), but is not limited thereto.

The electrolyte solution may have a pH of 5 to 12, preferably 7.4 to 10.

Further, the kit may include 4-dimethylaminopyridine at a concentration of more than 0 mM and 20 mM or less, more than 0 mM and 15 mM or less, more than 0 mM and 10 mM or less, or more than 0 mM and 7 mM or less.

As an exemplary embodiment of the present invention, an electrochemiluminescent label including a ruthenium compound is used to label an antigen or antibody, and electrochemiluminescent immunoassays by immunoreaction and ECL reaction may be performed. For the specific chemiluminescent reaction which is induced by electrochemistry at the surface of an electrode, an antibody (Ab) is labeled with an electrochemiluminescent reagent ruthenium pyridine and a carrier is coated with an antigen or antibody which forms a complex with the corresponding antigen or antibody in the sample by a specific mode of immune response. Label-conjugated complexes are separated from free labels by a separation technique. The antigen (Ag) or antibody (Ab) may be quantitatively or qualitatively measured by the luminescence intensity of ruthenium pyridine at an electrode. The co-reactant pyridine derivative flows into an electrochemical cell and a voltage may be applied to initiate the ECL reaction.

The use of a kit for electrochemiluminescence immunoassay or molecular diagnosis according to the present invention may significantly reduce the amount of antigens and antibodies when applied to a diagnostic device. Since the ECL luminescence intensity according to the electrochemical reaction between the pyridine derivative according to the invention and ruthenium pyridine is 20 times greater than that using tripropylamine, it may be detected using a considerably small amount of antigen and antibody, and therefore, the amount of antibody may be efficiently reduced. In addition, kits or analytical devices may be relatively inexpensive.

Furthermore, the use of a kit for electrochemiluminescence immunoassay or molecular diagnosis according to the present invention may obtain greater detection signals and greater detection sensitivity for the analyte compared to using tripropylamine.

As described above, the pyridine derivative represented by Chemical Formula I according to the present invention co-reacts with ruthenium pyridine, which is an electrochemiluminescent label, to significantly increase the luminescence intensity, so that it can be widely applied to *in vitro* diagnostic devices such as immunoassay.

### EXEMPLARY EMBODIMENTS OF THE INVENTION

Hereinafter, preferred examples for helping the understanding of the present invention will be suggested. However, the following examples are only provided such that the present invention may be more easily understood, and the content of the present invention is not limited by the following examples.

### < Experimental Example > Common experiment and measurement method

Before starting the electrochemiluminescence measurement in the ECL detector of the Example, a cell was washed with a washing solution (ethanol and water and dried with N₂ gas). The surface of a working electrode was also polished with an alumina (0.05 µm) slurry, sonicated with a mixture of deionized water (DI) and ethanol (1 : 1 v/v) within 5 minutes, rinsed and dried with N₂ gas.

After cleaning, the electrochemical cell was filled with an electrolyte solution and attached to a photomultiplier tube (PMT). Then, a voltage was applied between the working electrode and the reference electrode by supplying power, and through this, a liquid sample of the cell was charged with a current predetermined by a control signal, and a reaction was initiated at the working electrode. ECL light generated at the working electrode passes through a photomultiplier tube (PMT), and the ECL light from the ECL response to the working electrode was sensed by an optical detector such as a photomultiplier tube (PMT) located above and adjacent to the electrochemical cell. Further, the main body is completely enclosed in a darkroom environment, not shown in a Markush drawing, which allows the photomultiplier tube (PMT) to receive the ECL light generated from the cell without external interference.

FIG. 1 is an exemplary embodiment illustrating an electrochemiluminescence system for performing electrochemiluminescence measurements in the uses according to the present invention, and for electrochemiluminescence measurement, a potentiostat is used to induce a reaction in an electrochemical cell, and the potentiostat and electrochemiluminescence measurement software may be run to measure the luminescence intensity of electrochemiluminescence.

Specifically, the present inventors investigated the performance and influential parameters of a newly discovered co-reactant, 4-DMAP, through experiments and compared its performance with those of the existing co-reactants TPrA and DBAE. The measurement of ECL luminescence was performed by supplying 7 mL of electrolyte to the cell, applying a potential to electrodes, and the intensity of ECL light was recorded using a photomultiplier tube (PMT).

Furthermore, an ECL detection method by potential control was performed by sweeping from 0 V to 1.6 V at a rate of 0.1 V/sec. The above voltage values were assigned between the working electrode (glassy carbon electrode, platinum electrode or gold electrode) and the reference electrode (Ag/AgCl or Ag/Ag⁺). Below is a list of all important parameters.

### <Example 1> Confirmation of electrochemical behavior of various electrochemiluminescence co-reactants

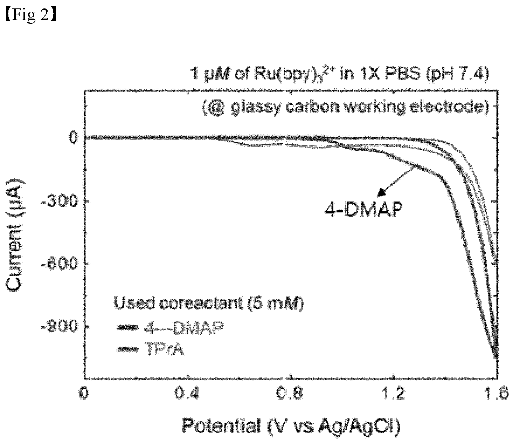

An electrochemiluminescent label Ru(bpy)₃²⁺, electrochemiluminescent co-reactants tripropylamine (TPrA), dimethyl ethanolamine (DBAE) and 4-dimethylaminopyridine (4-DMAP), and solvents phosphate buffer solution (PBS) and acetonitrile (ACN) were each prepared. These were mixed to prepare each sample, and a cyclic voltammogram (CV) and ECL intensity were measured and recorded for these samples. In this case, an experiment was performed using a potentiostat. A voltage scan started from 0.0 V at a rate of 0.1 V/s. In this case, a glassy carbon electrode, a platinum electrode and a silver electrode were used as working electrodes, and Ag/AgCl or Ag/AgNO₃ was used as a reference electrode. The upper voltage limit was 1.6 V, the lower voltage limit was 0 V, and the final voltage was 0 V. The electrochemical behavior of various electrochemiluminescence co-reactants was measured and shown in FIGS. 2 to 5.

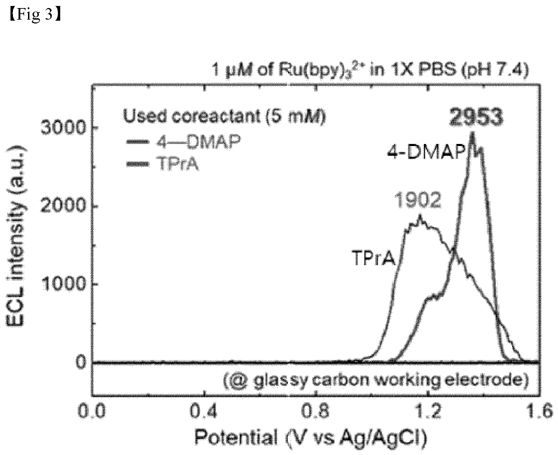

FIGS. 2 and 3 show the results of measuring the cyclic voltammogram (CV) and ECL intensity for the electrochemiluminescence system of Ru(bpy)₃²⁺/TPrA and Ru(bpy)₃²⁺/4-DMAP using glassy carbon materials as working electrodes during periodic potential scanning from 0 V to 1.6 V in PBS, respectively. As a result of FIGS. 2 and 3, it could be confirmed that the ECL intensity of Ru(bpy)₃²⁺/4-DMAP was higher than that of Ru(bpy)₃²⁺/TPrA.

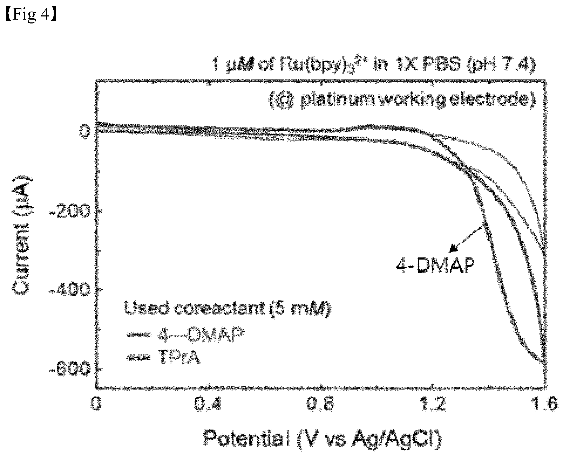

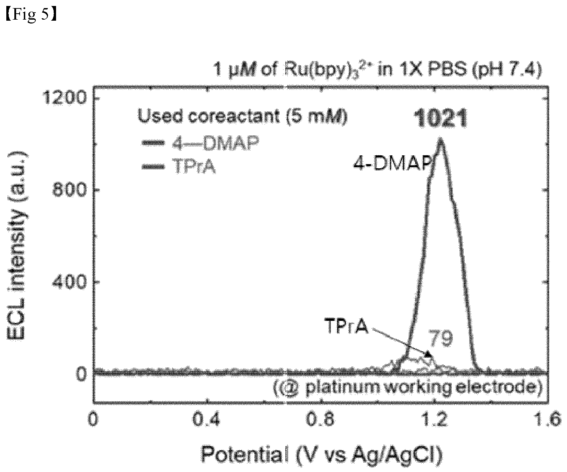

Meanwhile, FIGS. 4 and 5 show the results of measuring the cyclic voltammogram (CV) and ECL intensity for the electrochemiluminescence system of Ru(bpy)₃²⁺/TPrA and Ru(bpy)₃²⁺/4-DMAP using platinum materials as working electrodes during periodic potential scanning from 0 V to 1.6 V in PBS, respectively.

As a result of FIGS. 4 and 5, it could be confirmed that the anodic current of Ru(bpy)₃²⁺/4-DMAP was greater than that of Ru(bpy)₃²⁺/TPrA. Further, the ECL intensity of Ru(bpy)₃²⁺/4-DMAP was much greater than that of Ru(bpy)₃²⁺/TPrA.

Through these results, it could be confirmed that the platinum electrode was the most favorable working electrode for 4-DMAP oxidation in phosphate buffer saline (PBS), followed by the glassy carbon electrode.

### <Example 2> ECL characteristics according to concentration of electrochemiluminescence co-reactant

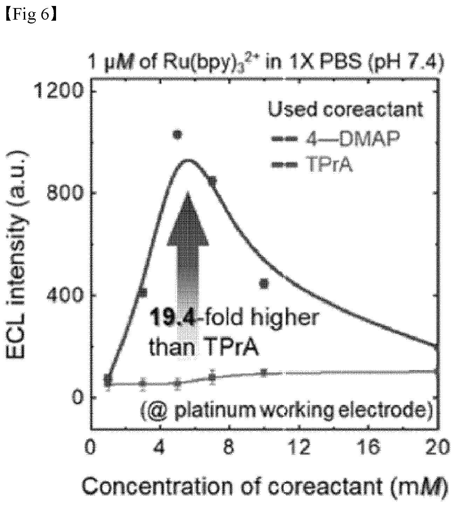

Sample solutions were prepared by varying the concentration of 4-DMAP, which is an electrochemiluminescence co-reactant, while maintaining the Ru(bpy)₃²⁺ concentration of the sample in PBS constant. Voltage scans were performed using the same procedure described in Example 1, and electrochemiluminescence intensities were measured in triplicate readings for each concentration of the electrochemiluminescence co-reactant. FIG. 6 illustrates the measurement results.

FIG. 6 shows the results of measuring the ECL intensity according to the concentration of 4-DMAP using a platinum electrode as a working electrode. It could be confirmed that the ECL intensity increased dramatically when 4-DMAP was used in a concentration range of more than 0 to 5 mM or less. However, it could be confirmed that the optimal concentration was 5 mM or less because the ECL intensity decreased when the 4-DMAP concentration was higher than 5 mM. Meanwhile, in the case of TPrA, it could be confirmed that the ECL intensity continued to increase when the TPrA concentration increased.

Through these results, it could be confirmed that when 4-DMAP was used as an electrochemiluminescence co-reactant, the concentration range of more than 0 mM and 5 mM or less provided the best luminescence intensity.

### <Example 3> ECL characteristics of electrochemiluminescence co-reactants according to various Ru(bpy)₃²⁺ concentrations

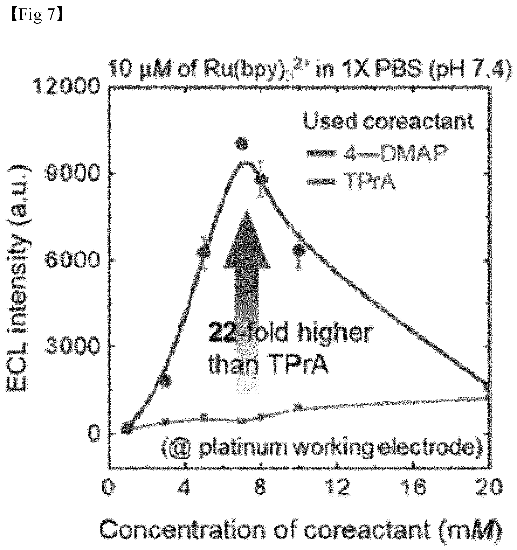

Samples of PBS including various concentrations of Ru(bpy)₃²⁺ were prepared. For voltage scans, the same procedure as described in Example 1 was used. The electrochemiluminescence intensity at each concentration of Ru(bpy)₃²⁺ was measured, and the results are shown in FIG. 7. As a result of FIG. 7, it could be confirmed that the intensity showed the highest intensity values during ECL measurements using a platinum (Pt) electrode as the working electrode at 10 Ru(bpy)₃²⁺ and a 7 mM 4-DMAP concentration, and the intensity decreased when the 4-DMAP concentration exceeded 7 mM.

Overall, it could be seen that the optimum concentration of 4-DMAP used as a co-reactant in a PBS solution including 10 µM Ru(bpy)₃²⁺ was around 7 mM.

### <Example 4> ECL characteristics of electrochemiluminescence co-reactant according to pH

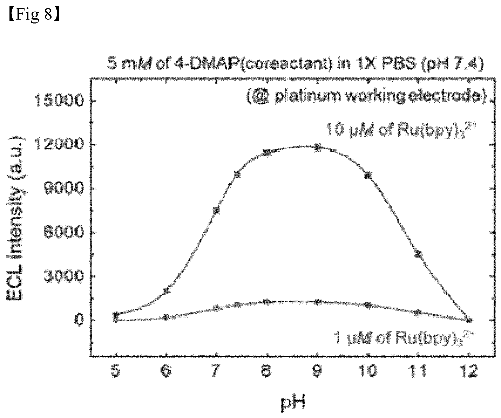

The present inventors attempted to confirm the ECL characteristics of the electrochemiluminescence co-reactant at various pH values. Electrochemiluminescence according to the electrochemiluminescence co-reactant was measured for PBS samples including constant concentrations of Ru(bpy)₃²⁺, except that the pH was varied from 5 to 12. Voltage scans were performed using the same procedure as described in Example 1. The electrochemiluminescence intensity was measured three times for different pH values, and the results are shown in FIG. 8.

FIG. 8 shows the results of examining 4-DMAP performance at different pH values, and it could be confirmed that a pH range from 7.4 to 10 was the most optimal range for 4-DMAP performance when 1 µM Ru(bpy)₃²⁺ and 5 mM 4-DMAP were used. Similarly, it could be observed that when 10 µM Ru(bpy)₃²⁺ and 7 mM 4-DMAP were used, the ECL intensity was inferior at a pH value of less than 7.4 and 10 or more. Therefore, it could be confirmed that the optimum pH was 7.4 to 10.

### <Example 5> ECL characteristics of electrochemiluminescence co-reactant under various solvents

Samples were prepared using an acetonitrile (ACN) solution instead of a PBS solution.

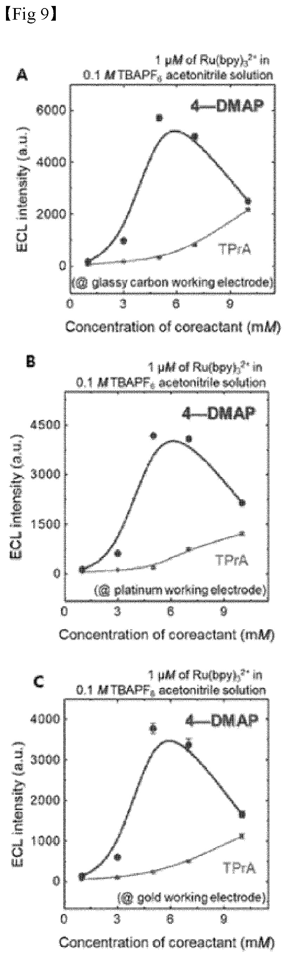

FIG. 9 shows the results of measuring the electrochemiluminescence intensity using an acetonitrile (ACN) solution instead of PBS. It could be confirmed that when a glassy carbon electrode (FIG. 9A), a platinum electrode (FIG. 9B) and a gold electrode (FIG. 9C) were each used as a working electrode, the ECL intensities of Ru(bpy)₃²⁺/4-DMAP at relatively low concentrations of 3 mM, 5 mM and 7 mM were higher than those of Ru(bpy)₃²⁺/TPrA.

### <Example 6> ECL characteristics of electrochemiluminescence co-reactant according to various luminescence materials

Each sample was prepared by mixing, as electrochemiluminescent labels, iridium-based transition metal complex compounds iridium bis[5-(trifluoromethyl)-2-(4-(trifluoromethyl)phenyl)pyridine] picolinate {(Ir(ppy-(CF₃)₂)₂(pico)}, bis(2-phenylpyridine)(4,4'-di-tert-butyl-2,2'-dipyridyl)iridium(III) (hexafluorophosphate) [Ir(dtbbpy)(ppy)₂PF₆] and Tris[2-(p-tolyl)pyridine]iridium(III) {Ir(mppy)₃}, 4-dimethylaminopyridine (4-DMAP), and a supporting electrolyte tetrabutylammonium hexafluorophosphate (TBAPF₆) with acetonitrile. Since the luminescence characteristics of these samples are very sensitively affected by oxygen and moisture, measurements were performed under a glove box in a nitrogen environment.

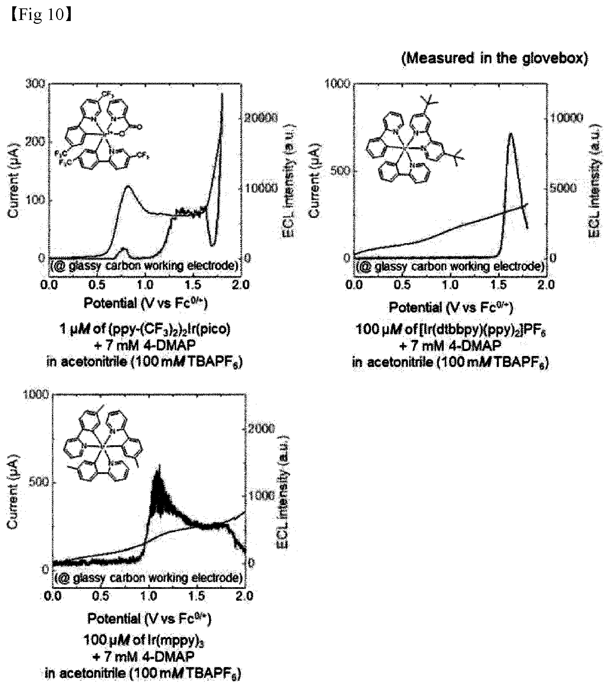

A linear sweep voltammogram (LSV) and ECL were measured and recorded for these samples. The scan rate started from 0.0 V at a rate of 0.1 V/s, and samples were scanned in a voltage range within 2.0 V. In this case, a glassy carbon electrode was used as a working electrode, Ag/Ag⁺ (3M AgNO₃) was used as a reference electrode, and Pt was used as an auxiliary electrode, and the results are shown in FIG. 10.

FIG. 10 shows the results of showing a linear sweep voltammogram and electrochemiluminescence intensity when 4-DMAP as a co-reactant and an iridium-based transition metal complex compound were used in an acetonitrile (ACN) solution, and glassy carbon was used as a working electrode. As a result of FIG. 10, it was confirmed that even when the iridium-based transition metal complex compound was used as an electrochemiluminescent label, the ECL intensity was shown to be high and the anodic current was also high.

Through these results, it can be seen that it is possible to use various types of transition metal complex compounds as electrochemiluminescent labels.

### <Example 7> Comparison of ECL detection curves according to concentration of Ru(bpy)₃²⁺ using electrochemiluminescence co-reactant

A PBS solution including a co-reactant at a concentration of 7 mM was prepared, and electrochemiluminescence signals generated when the concentration of Ru(bpy)₃²⁺ was changed were observed. Voltage scans were taken using the same procedure as described in Example 1. Electrochemiluminescence intensity was measured in triplicate readings under each condition.

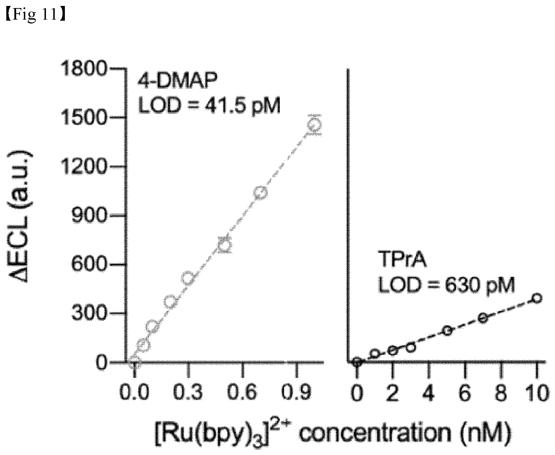

FIG. 11 shows the electrochemiluminescence intensity generated according to the concentration of Ru(bpy)₃²⁺ in each solution using 4-DMAP and tripropylamine as co-reactants. In the case of the tripropylamine co-reactant (right, black graph), electrochemiluminescence detection signals could be obtained for Ru(bpy)₃²⁺ in a concentration range of 0 to 10 nM, and the limit of detection for Ru(bpy)₃²⁺ was shown to be 0.63 nM (630 pM). In contrast, 4-DMAP shows a high electrochemiluminescence signal for Ru(bpy)₃²⁺ at a concentration of 0 to 0.1 nM, and the limit of detection was 0.0415 nM (41.5 pM), which shows a detection sensitivity at least 15 times greater than tripropylamine.

Through these results, it can be seen that it is possible to provide an electrochemiluminescence signal for the luminophore at a less concentration than tripropylamine, which can provide better detection sensitivity when used for immunodiagnosis or molecular diagnosis.

### <Example 8> Comparison of ECL detection curves according to concentration of Ru(bpy)₃²⁺ using electrochemiluminescence co-reactant

Chemiluminescent immunodiagnosis was performed on a "severe acute respiratory syndrome (SARS-CoV-2) neutralizing antibody (anti-SARS-CoV-2)" present in human saliva using 4-DMAP and tripropylamine as co-reactants, respectively. Saliva samples of ten vaccinated persons were collected and a total of three steps of electrochemical immunoassays were performed. Step 1) 30 µL of supernatant of centrifuged human saliva is mixed with a magnetic bead (2 µm diameter) reagent (capture reagent) to which a pre-prepared SARS-CoV-2 antigen was attached. The neutralizing antibody present in human saliva in this step forms a sandwich immunoconjugate with an antigen previously immobilized on magnetic beads. ii) After incubating this mixture for 30 minutes (37°C), only magnetic beads are collected and washed twice with PBS. Then, the beads are mixed with 30 µL of a 5 µg/mL human IgG antibody reagent (ECL signal generating reagent) to which a Ru(bpy)₃²⁺ label is attached. iii) Finally, only the magnetic beads are collected from the mixture of ii) above and captured on gold-printed electrodes. Electrochemiluminescence signals were measured by dropping 7 mM MDMAP or a 7 mM tripropylamine buffer solution (PBS) on the magnetic beads on the surface of the electrode, and applying a voltage to each.

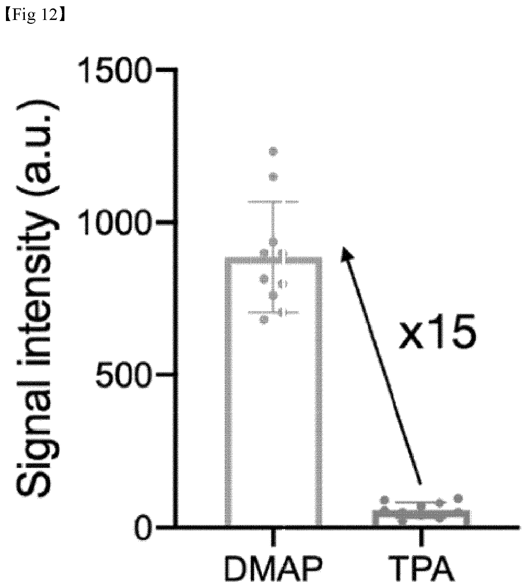

FIG. 12 illustrates the intensities of the electrochemiluminescence signals measured when 4-DMAP was used as a co-reactant and tripropylamine was used as a co-reactant in electrochemical immunodiagnosis for anti-SARS-CoV-2 neutralizing antibodies using saliva samples from 10 people inoculated with a vaccine. As a result, it could be seen that when 4-DMAP was used as a co-reactant for the same 10 saliva samples, detection signals appeared to be at least 15 times greater than tripropylamine.

Through this, it can be seen that greater detection sensitivity can be obtained when 4-DMAP is used as a co-reactant in electrochemiluminescence immunodiagnosis or molecular diagnosis.

Therefore, it could be confirmed that the luminescence intensity between the pyridine derivative represented by Chemical Formula I according to the present invention and ruthenium pyridine is 20-fold or greater than that obtained using tripropylamine, indicating that luminescence sensitivity is excellent. Therefore, since the pyridine derivative represented by Chemical Formula I according to the present invention can replace tripropylamine which is a co-reactant in the related art, and the voltage for light emission can be controlled to improve luminescence efficiency, it is possible to provide wide application potential in various bioanalyses such as immunoassays.

Although specific parts of the present invention have been described in detail, it is obvious to those skilled in the art that such specific descriptions are only preferred embodiments and the scope of the present invention is not limited thereby. The scope of the present invention is defined by the appended claims.

## Claims

1. Use of a compound represented by the following Chemical Formula I, or a pharmaceutically acceptable salt thereof, as an electrochemiluminescence co-reactant: 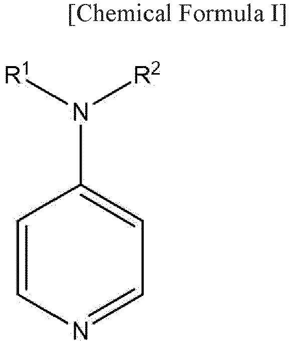 In Chemical Formula I, wherein each R¹ and R² are the same as or different from each other, and represent one selected from the group consisting of: a hydrogen atom; a halogen atom; a C₁-C₆ straight-chain, branched, or cyclic alkyl group; a C₁-C₆ alkoxy group; and a C₁-C₆ haloalkyl group.

2. The use of claim 1, wherein each R¹ and R² are the same as or different from each other, and are a C₁-C₄ straight-chain or branched alkyl group; or a C₁-C₄ haloalkyl group.

3. The use of claim 1, wherein Chemical Formula I is 4-dimethylaminopyridine (4-DMAP).

4. Use of an electrochemiluminescence system comprising: an electrochemical cell filled with an electrolyte solution comprising the compound or pharmaceutically acceptable salt of any one of claims 1 to 3 and an electrochemiluminescent label; and a photodetector connected to the electrochemical cell; wherein the use of the electrochemiluminescence system comprises using the compound or pharmaceutically acceptable salt as an electrochemiluminescence co-reactant.

5. The use of claim 4, wherein the electrochemical cell comprises a working electrode of one or more selected from the group consisting of carbon, platinum (Pt), gold (Au), silver (Ag), nickel (Ni), stainless steel, palladium, tin, indium and silicon elements.

6. The use of claim 4, wherein the electrolyte solution is one or more selected from the group consisting of phosphate buffer saline (PBS), acetonitrile (ACN), dichloromethane, ethanol, methanol, tetrahydrofuran (THF), dimethyl sulfoxide (DMSO), dimethylformamide (DMF), ethylene carbonate (EC) and propylene carbonate (PC).

7. The use of claim 4, wherein the electrolyte solution has a pH of 5 to 12.

8. The use of claim 4, wherein the electrochemiluminescent label is one or more selected from the group consisting of a transition metal complex compound, a luminescent organic semiconductor, a quantum dot material, perovskite nanoparticles, metal nanoparticles and carbon nanoparticles.

9. A detection method of an electrochemiluminescence system, the method comprising: (a) putting an electrolyte solution comprising a sample into an electrochemical cell in an electrochemiluminescence system as defined in claim 4 and reacting the resulting mixture, wherein a compound or pharmaceutically acceptable salt as defined in any one of claims 1 to 3 is used as an electrochemiluminescence co-reactant; and (b) measuring the electrochemiluminescence intensity (ECL intensity) according to the input voltage of the reaction sample of Step (a) using an electrochemiluminescence-based detector and detecting an optical signal.

10. Use of a kit for electrochemiluminescence immunoassay or molecular diagnosis, the kit comprising the compound or pharmaceutically acceptable salt as defined in any one of claims 1 to 3 and an electrochemiluminescent label; wherein the use of the kit comprises using the compound or pharmaceutically acceptable salt as an electrochemiluminescence co-reactant.

11. The use of claim 10, wherein the kit comprises an electrolyte solution of one or more selected from the group consisting of phosphate buffer saline (PBS), acetonitrile (ACN), dichloromethane, ethanol, methanol, tetrahydrofuran (THF), dimethyl sulfoxide (DMSO), dimethylformamide (DMF), ethylene carbonate (EC) and propylene carbonate (PC).

12. The use of claim 11, wherein the electrolyte solution has a pH of 5 to 12.

13. The use of claim 10, wherein the kit comprises 4-dimethylaminopyridine at a concentration of greater than 0 mM and 20 mM or less.

## Patentansprüche

1. Verwendung einer durch die folgende chemische Formel I wiedergegebenen Verbindung oder eines pharmazeutisch unbedenklichen Salzes davon als Elektrochemilumineszenz-Coreaktant: 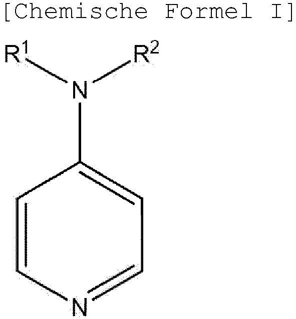 wobei in der chemischen Formel I R¹ und R² jeweils gleich oder voneinander verschieden sind und für eines aus der Gruppe bestehend aus einem Wasserstoffatom; einem Halogenatom; einer geradkettigen, verzweigten oder cyclischen C₁-C₆-Alkylgruppe; einer C₁-C₆-Alkoxygruppe und einer C₁-C₆-Halogenalkylgruppe stehen.

2. Verwendung nach Anspruch 1, wobei R¹ und R² jeweils gleich oder verschieden voneinander sind und für eine geradkettige oder verzweigte C₁-C₄-Alkylgruppe oder eine C₁-C₄-Halogenalkylgruppe stehen.

3. Verwendung nach Anspruch 1, wobei es sich bei der Chemischen Formel I um 4-Dimethylaminopyridin (4-DMAP) handelt.

4. Verwendung eines Elektrochemilumineszenzsystems, umfassend: eine elektrochemische Zelle, die mit einer Elektrolytlösung gefüllt ist, die die Verbindung oder das pharmazeutisch unbedenkliche Salz nach einem der Ansprüche 1 bis 3 und einen elektrochemilumineszierenden Marker umfasst; und einen Photodetektor, der mit der elektrochemischen Zelle verbunden ist; wobei die Verwendung des Elektrochemilumineszenzsystems die Verwendung der Verbindung oder des pharmazeutisch unbedenklichen Salzes als einen Elektrochemilumineszenz-Coreaktant umfasst.

5. Verwendung nach Anspruch 4, wobei die elektrochemische Zelle eine Arbeitselektrode aus einem oder mehreren aus der Gruppe bestehend aus Kohlenstoff-, Platin(Pt)-, Gold(Au)-, Silber(Ag)-, Nickel (Ni)-, Edelstahl-, Palladium-, Zinn-, Indium- und Siliciumelementen umfasst.

6. Verwendung nach Anspruch 4, wobei es sich bei der Elektrolytlösung um eines oder mehrere aus der Gruppe bestehend aus phosphatgepufferter Kochsalzlösung (PBS), Acetonitril (ACN), Dichlormethan, Ethanol, Methanol, Tetrahydrofuran (THF), Dimethylsulfoxid (DMSO), Dimethylformamid (DMF), Ethylencarbonat (EC) und Propylencarbonat (PC) handelt.

7. Verwendung nach Anspruch 4, wobei die Elektrolytlösung einen pH-Wert von 5 bis 12 aufweist.

8. Verwendung nach Anspruch 4, wobei es sich bei dem elektrochemilumineszierenden Marker um eines oder mehrere aus der Gruppe bestehend aus einer Übergangsmetallkomplexverbindung, einem lumineszierenden organischen Halbleiter, einem Quantenpunktmaterial, Perowskit-Nanopartikeln, Metall-Nanopartikeln und Kohlenstoff-Nanopartikeln handelt.

9. Detektionsverfahren eines Elektrochemilumineszenzsystems, wobei das Verfahren Folgendes umfasst: (a) Einbringen einer Elektrolytlösung, die eine Probe umfasst, in eine elektrochemische Zelle in einem Elektrochemilumineszenzsystem gemäß Anspruch 4 und Umsetzen der resultierenden Mischung, wobei eine Verbindung oder ein pharmazeutisch unbedenkliches Salz gemäß einem der Ansprüche 1 bis 3 als Elektrochemilumineszenz-Coreaktant verwendet wird; und (b) Messen der Elektrochemilumineszenzintensität (ECL-Intensität) gemäß der Eingangsspannung der Reaktionsprobe aus Schritt (a) unter Verwendung eines elektrochemilumineszenzbasierten Detektors und Detektieren eines optischen Signals.

10. Verwendung eines Kits für einen Elektrochemilumineszenz-Immunoassay oder eine molekulare Diagnose, wobei das Kit die Verbindung oder das pharmazeutisch unbedenkliche Salz gemäß einem der Ansprüche 1 bis 3 und einen elektrochemilumineszierenden Marker umfasst; wobei die Verwendung des Kits das Verwenden der Verbindung oder des pharmazeutisch unbedenklichen Salzes als Elektrochemilumineszenz-Coreaktant umfasst.

11. Verwendung nach Anspruch 10, wobei das Kit eine Elektrolytlösung von einem oder mehreren aus der Gruppe bestehend aus phosphatgepufferter Kochsalzlösung (PBS), Acetonitril (ACN), Dichlormethan, Ethanol, Methanol, Tetrahydrofuran (THF), Dimethylsulfoxid (DMSO), Dimethylformamid (DMF), Ethylencarbonat (EC) und Propylencarbonat (PC) umfasst.

12. Verwendung nach Anspruch 11, wobei die Elektrolytlösung einen pH-Wert von 5 bis 12 aufweist.

13. Verwendung nach Anspruch 10, wobei das Kit 4-Dimethylaminopyridin in einer Konzentration von mehr als 0 mM und 20 mM oder weniger umfasst.

## Revendications

1. Utilisation d'un composé représenté par la formule chimique suivante I, ou d'un sel pharmaceutiquement acceptable de celui-ci, en tant que coréactif d'électrochimioluminescence : 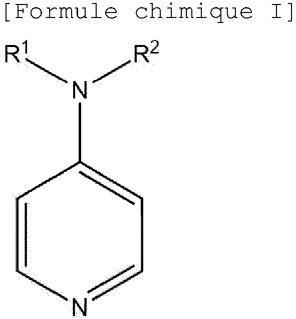 dans la formule chimique I, dans laquelle chaque R¹ et R² sont identiques ou différents l'un de l'autre, et représentent l'un choisit dans le groupe constitué par : un atome d'hydrogène ; un atome d'halogène ; un groupe alkyle linéaire, ramifié ou cyclique en C₁-C₆ ; un groupe alcoxy en C₁-C₆ ; et un groupe halogénoalkyle en C₁-C₆.

2. Utilisation selon la revendication 1, dans laquelle chaque R¹ et R² sont identiques ou différents l'un de l'autre, et sont un groupe alkyle en C₁-C₄ linéaire ou ramifié ; ou un groupe halogénoalkyle en C₁-C₄.

3. Utilisation selon la revendication 1, dans laquelle la formule chimique I est la 4-diméthylaminopyridine (4-DMAP).

4. Utilisation d'un système d'électrochimioluminescence comprenant : une cellule électrochimique remplie avec une solution d'électrolyte comprenant le composé ou sel pharmaceutiquement acceptable selon l'une quelconque des revendications 1 à 3 et un marqueur électrochimioluminescent ; et un photodétecteur connecté à la cellule électrochimique ; dans laquelle l'utilisation du système d'électrochimioluminescence comprend l'utilisation du composé ou sel pharmaceutiquement acceptable comme coréactif d'électrochimioluminescence.

5. Utilisation selon la revendication 4, dans laquelle la cellule électrochimique comprend une électrode de travail d'un ou plusieurs éléments choisis dans le groupe constitué par les éléments carbone, platine (Pt), or (Au), argent (Ag), nickel (Ni), acier inoxydable, palladium, étain, indium et silicium.

6. Utilisation selon la revendication 4, dans laquelle la solution d'électrolyte est une ou plusieurs solutions choisies dans le groupe constitué par une solution saline de tampon phosphate (PBS), l'acétonitrile (ACN), le dichlorométhane, l'éthanol, le méthanol, le tétrahydrofurane (THF), le diméthylsulfoxyde (DMSO), le diméthylformamide (DMF), le carbonate d'éthylène (EC) et le carbonate de propylène (PC).

7. Utilisation selon la revendication 4, dans laquelle la solution d'électrolyte a un pH de 5 à 12.

8. Utilisation selon la revendication 4, dans laquelle le marqueur électrochimioluminescent est un ou plusieurs marqueurs choisis dans le groupe constitué par un composé de complexe de métal de transition, un semi-conducteur organique luminescent, un matériau à point quantique, des nanoparticules de perovskite, des nanoparticules métalliques et des nanoparticules de carbone.

9. Procédé de détection d'un système d'électrochimioluminescence, le procédé comprenant : (a) le placement d'une solution d'électrolyte comprenant un échantillon dans une cellule électrochimique dans un système d'électrochimioluminescence tel que défini dans la revendication 4 et la mise en réaction du mélange résultant, dans lequel un composé ou un sel pharmaceutiquement acceptable tel que défini dans l'une quelconque des revendications 1 à 3 est utilisé comme coréactif d'électrochimioluminescence ; et (b) la mesure de l'intensité d'électrochimioluminescence (intensité ECL) en fonction de la tension d'entrée de l'échantillon réactionnel de l'étape (a) en utilisant un détecteur basé sur l'électrochimioluminescence et détectant un signal optique.

10. Utilisation d'un kit pour un dosage immunologique par électrochimioluminescence ou un diagnostic moléculaire, le kit comprenant le composé ou sel pharmaceutiquement acceptable tel que défini dans l'une quelconque des revendications 1 à 3 et un marqueur électrochimioluminescent ; dans laquelle l'utilisation du kit comprend l'utilisation du composé ou d'un sel pharmaceutiquement acceptable en tant que coréactif d'électrochimioluminescence.

11. Utilisation selon la revendication 10, dans laquelle le kit comprend une solution d'électrolyte d'un ou plusieurs éléments choisis dans le groupe constitué par une solution saline de tampon phosphate (PBS), l'acétonitrile (ACN), le dichlorométhane, l'éthanol, le méthanol, le tétrahydrofurane (THF), le diméthylsulfoxyde (DMSO), le diméthylformamide (DMF), le carbonate d'éthylène (EC) et le carbonate de propylène (PC).

12. Utilisation selon la revendication 11, dans laquelle la solution d'électrolyte a un pH de 5 à 12.

13. Utilisation selon la revendication 10, dans laquelle le kit comprend de la 4-diméthylaminopyridine à une concentration supérieure à 0 mM et 20 mM ou moins.
